# EUROPEAN PATENT SPECIFICATION

(11) **EP 1 641 359 B1**
(45) Date of publication and mention of the grant of the patent: **20.06.2012**
(21) Application number: 04742163.1
(22) Date of filing: 02.07.2004
(51) Int. Cl.: A23L 1/305, A23L 1/30, A23L 1/054, A23J 7/00, A61K 38/02, A61K 31/685, A61K 31/716, A61K 36/00

(54) **COMPOSITIONS AND FOOD PRODUCTS FROM OAT**
ZUSAMMENSETZUNGEN UND NAHRUNGSMITTEL AUF HAFERBASIS
COMPOSITIONS ET PRODUITS ALIMENTAIRES PREPARES A PARTIR D'AVOINE

(30) Priority: 03.07.2003 FI 20031004
(43) Date of publication of application: 05.04.2006
(73) Proprietor: Raisio Nutrition Ltd., 21201 Raisio (FI)
(72) Inventor: KUUSISTO, Päivi, FI-21100 Naantali (FI); WESTER, Ingmar, FI-20100 Turku (FI)
(74) Representative: Svensson, Johan Henrik
(86) International application number: PCT/FI2004/000420
(87) International publication number: WO 2005/002367

(56) References cited:
- EP-A- 0 619 950
- EP-A- 0 790 060
- WO-A-02/067698
- WO-A-03/055324
- US-A- 4 282 319
- US-A- 5 525 151
- US-A1- 2003 068 357
- ONNING G ET AL: "CONSUMPTION OF OAT MILK FOR 5 WEEKS LOWERS SERUM CHOLESTEROL AND LDL CHOLESTEROL IN FREE-LIVING MEN WITH MODERATE HYPERCHOLESTEROLEMIA" ANNALS OF NUTRITION AND METABOLISM, KARGER, CH, vol. 43, 2000, pages 301-309, XP008024693 ISSN: 0373-0101
- DATABASE WPI Section Ch, Week 198937 Derwent Publications Ltd., London, GB; Class B04, AN 1989-268301 XP002301245 & JP 01 196270 A (SNOW BRAND MILK PROD CO LTD) 8 August 1989 (1989-08-08) cited in the application

## Description

The present invention relates to the field of food and nutrition, especially to functional food. The new products concern therapeutical compositions and food products made from oat fractions. The compositions are suitable for improving the serum lipid profile. The invention is also related to methods for preparing the compositions.

### Background of the Invention

Cardiovascular disease (CVD) is one of the major causes of death in western world. Elevated serum total and/or LDL (low density lipoprotein) cholesterol and triacylglycerol levels, as well as a low ratio of HDL (high density lipoprotein) cholesterol to LDL cholesterol, are some of the major risk factors for CVD. Recently also serum levels of apolipoprotein B 100 (apo B) has been shown to be a reliable CVD risk maker. One of the first steps in improving the serum lipid profile is changes in life style including changes in diet and exercise. It seems, however, difficult to change dietary habits and to follow dietary recommendations. Thus, there is a clear need for solutions beyond regular diet and lifestyle changes by which serum lipid profile can be improved.

Food products enriched with components having cholesterol lowering effect beyond normal nutrition have been commercially available for some time. Representative examples of the components are soy protein and plant sterols, especially plant stanol or sterol fatty acid esters. Stanol fatty acid esters and the cholesterol lowering effects thereof are disclosed in US patent No. 5,502,045 as well as a suitable method for their preparation. Dietary intake of 2 to 3 g/day of plant stanols is reported to lower serum LDL cholesterol levels in man up to 14 % without affecting HDL cholesterol levels. FDA has also approved a health claim about the role of soy protein in reducing the risk of CVD by lowering blood cholesterol levels.

Also soy protein hydrolysate is known to lower serum cholesterol (JP 60011425). The cholesterol lowering effect of the soy protein hydrolysate might be attributed to certain peptides. Cholesterol lowering peptides have also been found from bovine milk β-lactoglobulin (Nagaoka et al., Biochem. Biophys. Res. Commun., 2001, 281(1), 11-17), but their efficacy still remains to be proven by well-controlled clinical studies.

EP 0 790 060 discloses a protein hydrolysate/phospholipid complex for improving lipid metabolism, where the complex contains 10 % or more bound phospholipids, especially lecithin or enzyme modified lecithin. EP 0 790 060 also discloses a process for preparing the protein hydrolysate/phospholipid complex which comprises mixing a protein hydrolysate with phospholipids and recovering the formed complex. Another process disclosed in EP 0 790 060 to prepare the protein hydrolysate/phospholipid complex comprises mixing the protein with a phospholipid to form a complex, hydrolysing the protein of the complex in an aqueous medium and recovering the formed protein hydrolysate/phospholipid complex.

Also oat bran and oat meal are known to have beneficial effects on serum cholesterol levels. The beneficial effect is attributed to the soluble fibre β-glucan. For example JP 1196270 describes processing of oat bran to increase the soluble fibre content and by increasing it, getting a higher serum lipid improving activity. β-glucan extracts are prepared from oats or other cereals such as barley. Typically the other components of oat, such as protein or lipids, are not regarded as valuable as β-glucan. The high lipid content of oats has been a major drawback when finding food applications to oat components, especially to oat protein. The oat fractions containing protein and lipids have mainly been used in animal feeds and cosmetics.

Hydrolysis, e.g. by enzymatic methods, has been used to obtain water soluble cereal proteins. WO 02/067698 uses starch and phytate hydrolysing enzymes in combination with aqueous wet milling for fractionation of cereal bran components. The bran is processed to obtain protein fractions derived substantially from the germ, residual endosperm or aleurone cells. Protein hydrolysate produced by incorporation of proteases into the above mentioned protein fractions in wet state, at controlled temperature and pH conditions, is also described. The protein hydrolysate has enhanced functionalities such as solubility, emulsifying and foaming capacities. These protein fractions and hydrolysates contain high amounts of soluble dietary fibre.

EP 0 619 950 describes a process to prepare a low fat, stable, water soluble oat protein fraction. Oat material is treated with protease outside the isoelectric range of the protein, preferably at alkaline conditions and insoluble fractions, i.e. insoluble solid fraction and insoluble liquid fraction which is primarily oat fat, are separated and the soluble oat protein fraction recovered. The protease treatment dissociates the soluble oat peptides and the oat fat. The small amount of oat fat remaining in the protein fraction after the alkaline treatment consists of free fatty acids. The obtained protein fraction contains therefore no emulsifying lipids. No therapeutical effects are attributed to these water soluble cereal protein hydrolysates.

US 4,282,319 discloses a process for preparing in situ enzymatically hydrolysed protein and starch products from whole cereal. The cereal material, such as wheat, barley or oat, is first subjected to an endopeptidase treatment in aqueous medium so as to transform substantially all water insoluble proteins present in the cereal to water soluble protein products, which thereafter are filtered and recovered as a clear filtrate. No therapeutical and/or health effects are attributed to these water soluble cereal protein hydrolysates.

US 2003/068357 A1 discloses a food product for lowering serum cholesterol levels, the active components being a soluble fiber and a sterol or a fatty acid derivative thereof. The soluble fiber may be psyllium, oat flour, oat bran, barley, beta-glucan, guar gum, beet pulp or pectin and the amount thereof is preferably between 1 and 25 percent by weight. The exemplified compositions also include protein.

Önning G. et al. "Consumption of Oat Milk for 5 Weeks Lowers Serum Cholesterol and LDL Cholesterol in Free-Living Men with Moderate Hypercholesterolemia", Annals of Nutrition and Metabolism, vol. 43, 2000, pages 301-309, deal with a study the aim of which was to investigate whether consumption of an oat milk deprived of insoluble fiber would result in lower serum cholesterol and LDL cholesterol levels in men. The tested oat milk was made from commercial oat flakes and a heat-treated, dry-milled oat bran fraction and in the manufacturing process the insoluble fibers were separated.

A combination of proteins and emulsifying lipids from an oat fraction as such or further treated with protease has not been associated with prophylactic and/or health effects, particularly not with serum lipid profile improving effect. The present invention provides new therapeutical compositions for improving the serum lipid profile i.e. for lowering the total and/or LDL cholesterol levels and/or lowering the apolipoprotein B levels and/or lowering the triglyceride levels and/or increasing the HDL cholesterol level and/or increasing the ratio of HDL to LDL cholesterol levels.

### Summary of the Invention

The present invention provides a therapeutical composition for oral use comprising an oat fraction comprising protein and naturally occurring emulsifying lipids. The therapeutical composition is prepared of an oat starting material containing both protein and naturally occurring emulsifying lipids. The composition contains at least 25 % by dry weight of proteins and at least 1 % by dry weight of emulsifying lipids.

The present invention also provides a therapeutical oat based composition for oral use comprising protein hydrolysate(s) and naturally occurring emulsifying lipids that has been prepared by hydrolysing an oat based starting material having both proteins and naturally occurring emulsifying lipids, preferably enzymatically with protease. The amount of protein hydrolysate is at least 15 % by dry weight and the amount of emulsifying lipids at least 1 % by dry weight in the therapeutical composition. In a preferred embodiment the composition is prepared of an oat fraction having increased protein content.

It has been found that oat based compositions comprising protein hydrolysates and naturally occurring emulsifying lipids can be used therapeutically, especially for serum lipid profile improving effect. The oat based compositions comprising protein hydrolysates and emulsifying lipids of this invention were compared to a commercial soy protein hydrolysate - phospholipid complex and surprisingly found to work in a similar or even in a better manner in the test. Also compositions comprising both protein and emulsifying lipids of the present invention worked in a corresponding way.

The present invention preferably utilises oat based fractions that are conventionally mostly used as feed raw materials for preparation of therapeutical compositions. Another advantage with the use of oats is that it is non-allergenic compared to e.g. both soy and wheat.

The therapeutical compositions of the present invention can be orally used as such or as pharmaceuticals or nutraceuticals or most advantageously in food products. Another aim of the invention is to provide a food product comprising the therapeutical compositions. The food products are preferably used for improving the serum lipid profile.

The invention also provides a method for preparing the therapeutical compositions using a starting material naturally comprising both a protein and emulsifying lipids. The preparation method of the compositions comprising protein hydrolysates includes hydrolysis of the protein. The emulsifying lipids remain with the resulting protein hydrolysate fraction. The obtained therapeutical composition contains at least 15 % by weight of protein hydrolysate and at least 1 % by weight of emulsifying lipids. The advantages of this method are a simplified and more cost effective preparation of the therapeutical compositions comprising protein hydrolysates and emulsifying lipids. No separate steps are needed to first separate and purify the protein and the lipid fractions, then prepare the protein hydrolysate and after that combine the protein hydrolysate and the lipid fraction.

One of the aims of the present invention is to provide a therapeutical composition for oral use obtained from oat fractions comprising protein and emulsifying lipids for improving serum lipid profile in humans and/or animals and a method for improving serum lipid profile in humans and/or animals by orally administering the composition.

Another aim of the present invention is to provide therapeutical oat based compositions containing protein hydrolysate(s) and emulsifying lipids for oral use for improving serum lipid profile in humans and/or animals and a method for improving serum lipid profile in humans and/or animals by orally administering the composition.

The characterizing features of the present invention are set forth in the claims.

### Detailed Description of the Invention

One aspect of the present invention is a therapeutical composition for oral use comprising at least 25 % by dry weight protein and at least 1 % by dry weight emulsifying lipids, both the protein and the emulsifying lipids being obtained from the same oat fraction. The weight ratio of protein to emulsifying lipids is from 1:0.01 to 1: 0.6 in the therapeutical composition. The composition further comprises β-glucan in an amount of at most 8 % by dry weight. The emulsifying lipids comprise phospholipids and glycolipids and the weight ratio of the phospholipids to glycolipids is from 1:0.7 to 1:6. The therapeutical composition is prepared of an oat starting material containing both protein and naturally occurring emulsifying lipids. The therapeutic composition comprises preferably at least 30 %, more preferably at least 35 % and most preferably at least 40 % by dry weight protein, and preferably at least 2 %, more preferably at least 2.5 %, and most preferably at least 3 % by dry weight emulsifying lipids.

Another aspect of the present invention is a therapeutical composition for oral use comprising at least 15 % by dry weight protein hydrolysate and at least 1 % by dry weight emulsifying lipids, both the protein and the emulsifying lipids being obtained from the same oat fraction which has been subjected to hydrolysis, and the weight ratio of protein hydrolysate to the emulsifying lipids is from 1:0.01 to 1:1. The composition further comprises β-glucan in an amount of at most 8 % by dry weight. The emulsifying lipids comprise phospholipids and glycolipids and the weight ratio of the phospholipids to glycolipids is from 1:0.7 to 1:6.

The invention is also directed to therapeutical oat based compositions for oral use comprising at least 15 % by dry weight protein hydrolysate(s) and at least 1 % by dry weight emulsifying lipids, both the protein and the emulsifying lipids being obtained from the same oat fraction which has been subjected to hydrolysis, preferably to enzymatic hydrolysis by using protease. The weight ratio of protein hydrolysate to emulsifying lipids is from 1:0.01 to 1:1. The composition further comprises β-glucan in an amount of at most 8 % by dry weight. The emulsifying lipids comprise phospholipids and glycolipids and the weight ratio of the phospholipids to glycolipids is from 1:0.7 to 1:6. In a preferred embodiment the compositions are prepared of oat fractions having increased protein content and containing emulsifying lipids. The preparation method includes hydrolysis of the protein. The emulsifying lipids remain with the resulting protein hydrolysate fraction.

As used here, the term "starting material" refers to an oat based material naturally comprising both protein and emulsifying lipids and used for the preparation of the compositions of the present invention. Also the term "oat fraction" is used for the starting material. The starting material contains at least 10 % by dry weight of protein and at least 1 % by dry weight of emulsifying lipids. The ratio of protein to emulsifying lipids in the starting material is preferably from 1:0.01 to 1:0.6. In a preferred embodiment, the starting materials are oat fractions with increased protein content and containing emulsifying lipids. In addition to the protein and the lipids having emulsifying properties, the starting material can also contain other components, such as other lipids, e.g. triglycerides, carbohydrates, such as fibre and starch, and various minor components. In another preferred embodiment the ratio of protein to emulsifying lipids is from 1:0.1 to 1:0.6, preferably from 1:0.15 to 1:0.6. The compositions of the present invention are characterised by that both the protein and the emulsifying lipids are obtained from the same oat fraction.

A preferred starting material is an oat endosperm-rich fraction comprising at least 10 %, preferably at least 20 % by dry weight protein and at least 1 %, preferably at least 2 %, more preferably at least 3 % by dry weight emulsifying lipids. Preferably an oat endosperm-rich fraction with increased protein content is used. It is technically easier to increase the protein content of an endosperm rich fraction than e.g. of fractions having higher content of cell wall polysaccharides, such as oat bran. An oat endosperm rich fraction is especially suitable for preparation of the compositions containing protein hydrolysate and emulsifying lipids because it is technically easier to produce these compositions from the oat endosperm rich fraction than e.g. from fractions such as oat bran having a higher content of cell wall polysaccharides, such as cellulose. The oat endosperm rich fraction is often used as feed raw material. Producing the therapeutical compositions of this invention thus also provides a new, more effective use for this fraction.

Another preferred starting material is a whole grain based oat fraction comprising at least 10 %, preferably at least 20 % by dry weight protein and at least 1 %, preferably at least 2 %, more preferably at least 3 % by dry weight emulsifying lipids. Preferably a whole grain based oat fraction with increased protein content is used. It is technically easier to increase the protein content of a whole grain based oat fraction than e.g. of fractions having higher content of cell wall polysaccharides, such as oat bran. Using a whole grain fraction as a starting material is also especially beneficial in a plant where no fractionation of the oat is available or fractionated raw material is not available.

Another preferred starting material is an oat bran-rich fraction comprising at least 10 %, preferably at least 20 % by dry weight protein and at least 1 %, preferably at least 2 %, more preferably at least 3 % by dry weight emulsifying lipids. Preferably an oat bran rich fraction with increased protein content is used. An oat bran rich fraction suits for the preparation of the compositions containing protein hydrolysate and emulsifying lipids because it has the highest protein and lipid content of all commercially produced oat fractions.

The starting material can be used for the preparation of the therapeutical compositions comprising protein or protein hydrolysates and naturally occurring emulsifying lipids either as such or as further processed. Typical examples of starting materials include whole grain oat flour, oat fractions such as endosperm rich flour or bran rich flour, and especially oat fractions with increased protein content, which means that the protein content is higher than the natural protein content in the part of the oat where the material is obtained from. The protein content may be increased by e.g. at least 30 %, preferably at least 50 %, more preferably at least 80 % of the natural protein content. For example, the protein content of oat endosperm flour, obtained by conventional milling techniques, is naturally about 11 % by weight. This flour may be treated, e.g. enzymatically or by other means to increase the protein content in the oat fraction to be used as starting material. The increased protein content may be obtained e.g. by removing part of the starch and/or other carbohydrates from the oat fraction to be used as starting material.

As used here "emulsifying lipids" refer to lipid substances naturally occurring in the starting material and promoting the formation and improving the stability of emulsions. The characterizing feature of the emulsifying lipids is a structure in which one portion of the molecule is polar (hydrophilic) and the other non-polar (hydrophobic), which allows the emulsifying lipid to align and stabilize the contact surfaces of the two immiscible phases of the emulsion. Having the adequate amount of the emulsifying lipids in the compositions of the present invention enhances the therapeutical effect of the compositions and ensures the optimal availability of the ingredients at the site of action and is therefore an essential feature of the invention. The emulsifying lipids also facilitate the incorporation of the compositions into food products. By using a starting material naturally containing emulsifying lipids, there is not necessary any need to add emulsifiers from a separate source into compositions of the present invention. Oat lipids, i.e. oat polar lipid fractions, containing phospholipids and glycolipids are excellent for the purpose of this invention. In the composition of the invention the emulsifying lipids comprise phospholipids and glycolipids, the weight ratio of phospholipids to glycolipids is from 1:0.7 to 1:6, preferably from 1:1 to 1:5 and more preferably from 1:1 to 1:3.

Besides proteins and emulsifying lipids the starting material comprises other grain based materials such as other lipids, carbohydrates and various minor components.

As used here, by the term "protein hydrolysate" is meant all hydrolysed products of proteins prepared by using a proteolytic enzyme preparation, a microorganism containing suitable proteolytic activity or acid hydrolysis or any combination thereof, and having the therapeutical effect.

Preferred protein hydrolysates have a molecular weight of 300 - 100 000 D, more preferably 500 - 50 000 D, still more preferably 500 - 30 000 D, even more preferably 900 - 30 000 D, and most preferably 1600 - 30 000 D and/or they are not soluble or only slightly soluble in water (at most 20 % by weight of the total protein hydrolysate in water at 25°C at neutral pH-conditions). A preferred protein hydrolysate is resistant protein, which is hydrolysed in a way that not more than 60 % by weight, preferably not more than 40 %, of the resistant protein is further digestible in humans.

The compositions comprising protein hydrolysates are prepared of the starting material by hydrolysing the protein. The emulsifying lipids remain with the resulting protein hydrolysate fraction. So the compositions comprising protein hydrolysates and emulsifying lipids are characterised by that both the protein and the emulsifying lipids are obtained from the same starting material, without first separating and then combining the protein and the emulsifying lipids.

The protein of the starting material is preferably hydrolysed by enzymatic methods and processes well known in the art. Typical preparation methods include treatment of the material containing protein and the emulsifying lipids with a proteolytic enzyme in aqueous medium. The starting material is dispersed in water and pH is adjusted with an acid or a base to the optimum pH range of the enzyme and the protein to be used. The enzyme is preferably added in an amount of 0.2 - 5 %, more preferably 0.5 - 2 % by weight based on the substrate protein and the reaction is carrier out at optimum temperature and pH for the time needed depending on the enzyme and the degree of hydrolysis wanted. Typically the degree of hydrolysis is from 1 to 40 %, preferably from 3 to 30 % and more preferably from 5 to 20 % (analysed as described by Nielsen et al., 2001, Journal of Food Science, vol. 66, no 5). The reaction is typically terminated by heating the mixture to a temperature high enough to inactivate the enzyme. The inactivation may also be accomplished by pH adjustment. The reaction mixture can optionally be neutralized before or after the heating step by using a suitable acid, e.g. hydrochloric acid, or a base, e.g. sodium hydroxide.

The composition can be used as such or the reaction mixture can be separated into fractions based e.g. on molecular weight or solubility into water. Most preferably the precipitate obtained e.g. by centrifugation or other corresponding separation techniques (a water insoluble or slightly water soluble fraction) or fraction obtained by ultra filtration is used. Most preferably the precipitate is washed with water to remove the water soluble components more efficiently. Also further process steps, such as further purification of certain peptide fractions or peptides and/or drying, can be included. For example freeze-drying, spray drying or any other drying processes known in the art, which produce a powder either directly or through a grinding step, may be used.

Proteolytic enzymes that can be used in the preparation of the protein hydrolysates of the present invention include enzymes from plant, microbial and animal origin, including also enzymes from genetically modified organisms and micro-organisms having proteolytic activity that at suitable reaction conditions hydrolyse proteins to the therapeutical hydrolysates of this invention. Suitable proteolytic enzymes are e.g. pepsin, microbial aspartic proteases, trypsin, pancreatin, papain, subtilisin and other microbial serine proteases, thermolysin and bromelain. It is also possible to use different enzymes sequentially, e.g. pepsin and trypsin or pepsin and pancreatin. Especially preferred are the enzymes or combinations of enzymes that produce protein hydrolysates that are only poorly digestible or no longer digestible in human intestine and are thus also called "resistant proteins" and that have the therapeutic effect of the present invention. Examples of these preferred enzymes include especially pepsin, trypsin and pancreatin.

A therapeutical composition according to the invention comprises at least 15 %, preferably at least 20 %, more preferably at least 30 % by dry weight protein hydrolysate and at least 1 %, preferably at least 2 %, more preferably at least 3 %, still more preferably at least 4 %, even more preferably at least 5 % and most preferably at least 6 % by dry weight emulsifying lipids, both the protein and the emulsifying lipids are obtained from the same oat fraction which has been hydrolysed, preferably treated with protease, and the weight ratio of protein to the emulsifying lipids is from 1:0.01 to 1:1.

In a preferred therapeutical composition comprising protein hydrolysate and emulsifying lipids, the protein hydrolysate content is from 15 to 99 %, preferably from 20 to 98 %, more preferably from 30 to 97 %, most preferably from 30 to 94 % by dry weight of the therapeutical composition and the content of the emulsifying lipids is from 1 to 15 %, preferably from 2 to 15 %, more preferably from 2.5 to 15 %, still more preferably from 3 to 15 %, even more preferably from 4 to 15 %, still even more preferably from 5 to 15 %, and most preferably from 6 to 15 % by dry weight of the therapeutical composition. The ratio of the protein hydrolysate to the emulsifying lipids is from 1:0.01 to 1:1, preferably from 1:0.02 to 1:0.5 and most preferably from 1:0.03 to 1:0.3. The rest of the composition, i.e. from 0 to 84 %, preferably from 0 to 78 %, more preferably from 0 to 67 % and most preferably from 0 to 64 % by dry weight, may comprise compounding agents and/or grain based materials such as carbohydrates, lipids other than emulsifying lipids and various minor components.

In another preferred therapeutical composition comprising protein hydrolysate and emulsifying lipids, the protein hydrolysate content is from 15 to 98 %, preferably from 20 to 98 %, more preferably from 30 to 97 % and most preferably from 30 to 94 % by dry weight of the therapeutical composition and the content of emulsifying lipids is from 2 to 15 %, preferably from 2 to 10 %, more preferably from 2.5 to 10 %, still more preferably from 3 to 8 %, even more preferably from 4 to 8 %, still even more preferably from 5 to 8 % and most preferably from 6 to 8 % by dry weight of the therapeutical composition. The ratio of the protein hydrolysate to the emulsifying lipids is from 1:0.02 to 1:1, preferably from 1:0.02 to 1:0.5 and most preferably from 1:0.03 to 1:0.3. The rest of the composition, i.e. from 0 to 84 %, preferably from 0 to 78 %, more preferably from 0 to 67 % and most preferably from 0 to 64 % by dry weight, may comprise compounding agents and/or grain based materials such as carbohydrates, lipids other than emulsifying lipids and various minor components.

In a preferred therapeutical composition comprising protein hydrolysate and emulsifying lipids the dietary fibre content is at most 15 %, preferably at most 10 %, more preferably at most 5 % and most preferably at most 3 % by dry weight.

In another preferred therapeutical composition comprising protein hydrolysate and emulsifying lipids the β-glucan content is at most 5 %, preferably at most 3 % and more preferably at most 2 % by dry weight.

A therapeutical composition according to the invention comprises at least 25 %, preferably at least 30 %, more preferably at least 35 %, and most preferably at least 40 % by dry weight protein and at least 1 %, preferably at least 2%, more preferably at least 3 %, still more preferably at least 4 %, even more preferably at least 5 % and most preferably at least 6 % by dry weight emulsifying lipids, and the weight ratio of protein to emulsifying lipids is from 1:0.01 to 1:0.6.

In a preferred therapeutical composition comprising protein and emulsifying lipids, the protein content is from 25 to 99 %, preferably from 30 to 98 %, more preferably from 35 to 97.5 %, still more preferably from 40 to 97 % and most preferably from 40 to 94 % by dry weight of the therapeutical composition and the content of the emulsifying lipids is from 1 to 15 %, preferably from 2 to 15 %, more preferably from 2.5 to 15 %, still more preferably from 3 to 15 %, even more preferably from 4 to 15 %, still even more preferably from 5 to 15 % and most preferably from 6 to 15 % by dry weight of the therapeutical composition. The ratio of the protein to the emulsifying lipids is from 1:0.01 to 1:0.6, preferably from 1:0.03 to 1:0.6, more preferably from 1:0.1 to 1:0.6, still more preferable from 1:0.15 to 1:0.6, and most preferably from 1:0.15 to 1:0.4. The rest of the composition, i.e. from 0 to 74 %, preferably from 0 to 68 %, more preferably from 0 to 62.5 %, still more preferably from 0 to 57 % and most preferably from 0 to 54 % by dry weight, may comprise compounding agents and/or grain based materials such as carbohydrates, lipids other than emulsifying lipids and various minor components.

In another preferred therapeutical composition comprising protein and emulsifying lipids, the protein content is from 25 to 98 %, preferably from 30 to 98 %, more preferably from 35 to 97.5 %, still more preferably from 40 to 97 % and most preferably from 40 to 94 % by dry weight of the therapeutical composition and the content of the emulsifying lipids is from 2 to 15 %, preferably from 2 to 10 %, more preferably from 2.5 to 10 %, still more preferably from 3 to 8 %, even more preferably from 4 to 8 %, still even more preferably from 5 to 8 % and most preferably from 6 to 8 % by dry weight of the therapeutical composition. The ratio of the protein to the emulsifying lipids is from 1:0.02 to 1:0.6, preferably from 1:0.03 to 1:0.6, more preferably from 1:0.05 to 1:0.6 and most preferably from 1:0.1 to 1:0.4. The rest of the composition, i.e. from 0 to 74 %, preferably from 0 to 68 %, more preferably from 0 to 62.5 %, still more preferably from 0 to 57 % and most preferably fro 0 to 54 % by dry weight, may comprise compounding agents and/or grain based materials such as carbohydrates, lipids other than emulsifying lipids and various minor components.

In a preferred therapeutical composition comprising protein and emulsifying lipids, the dietary fibre content is at most 15 %, preferably at most 10 %, more preferably at most 5 % and most preferably at most 3 % by dry weight.

In another preferred therapeutical composition comprising protein and emulsifying lipids, the β-glucan content is at most 5 %, preferably at most 3 % and more preferably at most 2 % by dry weight.

A preferred embodiment of the invention is the use of an oat endosperm-rich fraction as a source for the protein hydrolysate and emulsifying lipids, whereby technically easily and cost-effectively a functionally desirable therapeutical composition as disclosed in this specification is obtained.

The therapeutical effects of the compositions of the present invention are directed for improving serum lipid profile, especially in humans and/or animals. Thus in an aspect of the present invention there is provided a method for improving serum lipid profile e.g. for lowering total and/or LDL cholesterol and/or increasing HDL cholesterol and/or reducing triacylglycerol and/or reducing apolipoprotein B and/or increasing the ratio of HDL cholesterol to LDL cholesterol in a subject, comprising orally administering to the subject a therapeutical composition according to the present invention in an amount effective for improving serum lipid profile. Further, the present invention provides a method to reduce or prevent the development of atherosclerosis in humans by dietary means including orally administering to the subject a composition according to the present invention in an amount effective for improving serum lipid profile.

The present invention is also directed to ensuring optimal availability of the active ingredients, especially protein hydrolysates, at the site of action. Compositions containing protein hydrolysates and emulsifying lipids according to the current invention are especially beneficial as the lipid profile improving effect is optimized by ensuring optimal availability of the active ingredients.

In still another aspect of the present invention, there is provided a combination of plant sterols and the therapeutical oat based compositions comprising protein hydrolysate(s) and naturally occurring emulsifying lipids. There is also provided a combination of plant sterols and the therapeutical oat fraction comprising protein and naturally occurring emulsifying lipids. The weight ratio of plant sterol to protein hydrolysate/protein is from 1:0.02 to 1:150, preferably from 1:0.2 to 1:30, more preferably from 1:0.4 to 1:12.5 and most preferably from 1:1 to 1:5 in the therapeutical compositions. Also plant sterols improve serum lipid profile, especially lower serum total and LDL cholesterol levels, and enhance surprisingly well the serum lipid profile improving effects of the oat based compositions of the present invention. Plant sterols are defined e.g. in WO 02/082929 and include both unsaturated and saturated desmethyl-sterols in their free form, esterified e.g. with fatty acids and mixtures of these.

The composition of the present invention can be used as such or as a pharmaceutical or a nutraceutical or most advantageously in food products for improving serum lipid profile in man. The compositions can be added in any suitable way into food products, pharmaceuticals or nutraceuticals. Incorporation of the compositions into food products, pharmaceuticals or nutraceuticals can be facilitated by reducing particle size of said compositions e.g. by grinding, milling or micromilling. The active ingredients are added into food material, pharmaceuticals or nutraceuticals by conventional processes for producing these products.

In a further aspect of the present invention there is provided a food product comprising at least one basic nutritional ingredient and at least one of the therapeutical compositions of the present invention as defined above.

The food product of the present invention can be prepared by adding said composition to food material(s) by the conventional processes for producing food products. The composition can be added e.g. as in liquid, semi-solid or dried form.

The food product of the present invention can be in the form of various food compositions, including but not restricted to
- bakery products and confectionery (fresh and dry bakery products, e.g. fresh bread, other bread products, cakes, muffins, waffles, biscuits, crackers, cookies, protein enriched bakery products etc.)
- cereal products and snacks (breakfast cereals, muesli, bars, such as cereal based and muesli bars, such bars possibly containing chocolate, pasta products, flours etc.)
- beverages (alcoholic and non-alcoholic drinks, including e.g. soft drinks, juices and juice-type mixed drinks, fortified beverages such as e.g. protein or calcium fortified beverages, probiotic drinks, sports and energy drinks, dietary supplement and meal replacement drinks, concentrates or premixes for beverages and powdered drinks where the content of compositions of the present invention is calculated for the ready-to-use form, etc.)
- dairy products (milk, milk based products, e.g. cheese, cream cheese and the like, yogurt, frozen yogurt, other frozen dairy foods, drinkable yogurt, other fermented milk products, dairy beverages, creams such as coffee cream, ice cream, desserts, spreads etc.)
- fermented cereal products
- sauces, soups
- meat, fish, poultry and egg products (e.g. sausages, meat balls etc.)
- analogues for e.g. dairy or meat products (e.g. imitations of cheese, yogurt, ice cream, desserts, meat substitutes, milk alternatives etc.), non-dairy frozen desserts
- soy based products
- vegetable oil based products (e.g. margarines, spreads, dressings, mayonnaise etc.)
- ready mixes (for baking e.g. breads, cakes, muffins, waffles, pizzas, pancakes; or for cooking e.g. soups, sauces, desserts, puddings) to be used in preparing or manufacturing foods.

Solid and semi-solid food products are especially preferred. These products typically have a viscosity of at least 0.1 Pas, more typically at least 0.5 Pas measured by a Brookfield viscometer, at the temperature of conventional use of the product in question.

The food product of the present invention can also contain other nutritionally beneficial components, some of which may further enhance the effects of the compositions of the present invention. The food can be fortified with these components or the components can be an intrinsic part of the other food ingredients.

Examples of the nutritionally beneficial components include plant sterols (as described above), diacylglycerol and n-3 fatty acids that also have advantageous effects on the lipid metabolism and may thus reduce the risk of cardiovascular disease. Other non-limiting examples of the beneficial nutritional components include beneficial minor components, for example such as isoflavones, tocopherols, tocotrienols, carotenoids, vitamin C, folate and flavonoids. Also other vitamins and minerals may be added or included in the food products of the present invention.

In a further aspect of the present invention there is provided a pharmaceutical or nutraceutical product for improving serum lipid profile comprising a composition of the present invention as defined above. Said product can additionally contain at least one compounding agent. The compounding agent can be any pharmaceutically acceptable binder, carrier, diluent, excipient or coating agent. The product can be in any suitable form, e.g. tablets, granules, powder, capsules, syrups, dispersions or other liquid preparations for oral administration.

The present invention also relates to the use of a composition as defined above, i.e. a therapeutic composition comprising protein and/or protein hydrolysate and emulsifying lipids, both the protein/protein hydrolysate and the emulsifying lipids being obtained from the same oat fraction for the manufacture of a pharmaceutical or nutraceutical or food product for improving the serum lipid profile.

When the therapeutical composition comprising protein hydrolysate and emulsifying lipids according to the invention is added to a food product, the content of the composition is typically between 0.01-70 g per 100 g food product, preferably 0.1-60 g/100 g, more preferably 0.5-50 g/100 g, and most preferably 1.0-50 g/100 g.

The optimal daily intake of the therapeutical compositions comprising protein hydrolysate and emulsifying lipids of the present invention as such or when added to foods, nutraceuticals or pharmaceuticals is such that a daily intake of the protein hydrolysate and the emulsifying lipids of 0.1-60 g, preferably 0.5-20 g, more preferably 0.8-10 g is supplied.

When the therapeutical composition comprising protein and emulsifying lipids according to the invention is added to a food product, the content of the composition is typically between 0.01-70 g per 100 g food product, preferably 0.1 - 60 g/100 g, more preferably 1.0-50 g/100 g, and most preferably 2.0-40 g/ 100 g.

The optimal daily intake of the therapeutical compositions comprising an oat fraction comprising protein and emulsifying lipids of the present invention as such or when used in foods, nutraceuticals or pharmaceuticals is such that a daily intake of the protein and the emulsifying lipids of 0.5-100 g, preferably 1.5-75 g, more preferably 3.0-50 g is supplied.

The compositions according to the present invention are most preferably incorporated into foods designed for being a part of a healthy diet.

The present invention also provides a method for preparing a therapeutical composition as defined above comprising at least 25 % by dry weight protein and at least 1 % by dry weight emulsifying lipids, said method comprising treating an oat fraction comprising both protein and emulsifying lipids and carbohydrates, such as starch, to remove carbohydrates, preferably by enzymatic hydrolysis, and to obtain said therapeutic composition having an increased content of protein.

Additionally the present invention provides a method for preparing a therapeutical composition as defined above comprising at least 15 % by dry weight of protein hydrolysate and at least 1 % by dry weight of emulsifying lipids, said method comprising hydrolysing an oat fraction comprising both protein and emulsifying lipids to obtain said therapeutical composition. The oat fraction is preferably subjected to enzymatic hydrolysis, especially using protease. The degree of hydrolysis is preferably from 1 to 40 %, more preferably from 3 to 30 % and most preferably from 5 to 20 %.

The invention is further illustrated by the following examples. In this specification the percentages mean % by weight unless otherwise specified.

### Example 1

An oat endosperm rich fraction with increased protein content was used as a starting material for the preparation of a composition containing protein hydrolysates and emulsifying lipids.

Oat endosperm rich flour (bran removed by milling of whole grain oat), which contained 12 % protein and 7 % lipids, 64 % starch and 5 % dietary fibre (Raisio) was suspended into water (1:10) and treated with α-amylase (Fungamyl 800L, Novozymes) at 65°C for 1 hour to reduce the starch content. The reaction mixture was further treated with cellulase and β-glucanase at 55°C (GC440, Genencor and Ultraflo L, Novozymes, 2 % of the starting material) to hydrolyse the rest of the β-glucan, although most of the β-glucan is soluble in water at 65°C and could have been removed from the insoluble fraction by e.g. centrifugation. The hydrolysed products of starch and β-glucan were removed from the precipitate by centrifugation. The resulting precipitate (=endosperm rich fraction with increased protein content) was freeze dried and contained 31 % starch, 38 % protein and 14 % lipids in total, including >2 % emulsifying lipids.

The dried endosperm rich fraction was suspended in water (1:10), pH was adjusted to 2 with hydrochloric acid and the protein hydrolysed enzymatically by using pepsin (Sigma Aldrich, P7000, activity 1:10000, 1 % of the protein content of the oat flour). The reaction was carried out at 37°C, for 24 hours and then the enzyme was inactivated by heating the reaction mixture to 90°C for 30 min. The mixture was neutralized with sodium hydroxide and centrifuged (4500 x g). The precipitate was washed twice with water after which the composition was freeze dried. The composition contained 20 % protein hydrolysate and 14 % lipids in total, including >2 % emulsifying lipids.

### Example 2

Whole grain oat material (average composition 13 % protein, 7 % lipids in total, 67 % carbohydrates) was used as a starting material for preparation of a composition containing protein hydrolysate and emulsifying lipids. The whole grain starting material was first enzymatically processed to decrease the amount of starch and fibre and to obtain a composition having increased protein content and emulsifying lipids (27 % protein, 9 % lipids in total, including >2 % emulsifying lipids, and 52 % carbohydrates). This oat fraction with increased protein content was used for the preparation of the composition containing protein hydrolysates and emulsifying lipids.

The oat fraction was mixed into water (1:10) and hydrolysed with pepsin at pH 2 as in the example 1 until the inactivation step. The pH of the reaction mixture was adjusted to 5 and the mixture was heated to 90°C to inactivate the pepsin and to gelatinize the starch. During the inactivation step at 90°C, thermostable α-amylase (Spezyme FRED, Genencor, 1 % of the starting material) was added into the reaction vessel to further reduce the starch content of the material. α-amylase was allowed to react for 2 hours, after which the reaction mixture was cooled to 55°C and pH adjusted to 6. The fibre components were hydrolysed by treating the mixture with cellulase and β-glucanase at 55°C (GC440, Genencor and Ultraflo L, Novozymes, both 1 % of the starting material) after which the mixture was centrifuged.

The precipitate was washed once with water, after which the composition containing protein hydrolysate and emulsifying lipids was spray dried. The composition contained 33 % protein hydrolysate, 14 % lipids in total, including >3.0 % emulsifying lipids.

### Example 3

The oat fraction with increased protein content from example 5 (46 % protein and 12 % lipids, including >3 % emulsifying lipids) was used as a starting material for the preparation of a composition containing protein hydrolysates and emulsifying lipids.

The oat protein preparation was suspended in water (1:10) and the protein hydrolysed enzymatically by using a microbial protease from *Aspergillus niger* at neutral pH (1 % of the protein content of the oat protein preparation). The enzyme was inactivated by heating the reaction mixture to 90°C for 30 min. The mixture was centrifuged and the precipitate freeze dried.

### Example 4

The same oat fraction as in example 2 (27 % protein, 9 % lipids in total, including >2 % emulsifying lipids, and 52 % carbohydrates) was used for the preparation of a composition containing protein hydrolysates and emulsifying lipids.

The oat fraction was suspended into water (1:10) and first treated with α-amylase (Fungamyl 800L, Novozymes, 1 % of the starting material, 65°C, 1 hour) to partly hydrolyse the starch and then with cellulase and β-glucanase at 55°C for 2 hours (GC440, Genencor and Ultraflo L, Novozymes, both 2 % of the starting material) to hydrolyse the fibre components. The resulting mixture was then hydrolysed at 37°C with pepsin (Sigma, pH 2, 8 hours, 1 % of the protein content of the starting material) and with pancreatin (Sigma, pH 8, 8 hours, 1 % of the protein content of the starting material). The enzymes were inactivated by heating the reaction mixture to 90°C for 30 min. The mixture containing protein hydrolysate and emulsifying lipids was spray dried as such.

### Example 5

A therapeutical composition comprising an oat fraction comprising protein and emulsifying lipids was prepared of whole grain oat material (average composition 13 % protein, 7 % lipids in total, 67 % carbohydrates). The whole grain starting material was enzymatically processed in two stages to obtain a composition comprising protein and emulsifying lipids. First an oat material containing 29 % protein, 10 % lipids in total, including at least 2.5 % emulsifying lipids (by dry weight), was obtained.

This material with increased protein content was further processed: the oat material was suspended in water and heated to 90°C to gelatinize the starch. A thermostable α-amylase (Spezyme FRED, Genencor, 1 % of the starting material) was added into the reaction vessel and allowed to react for 2 hours to reduce the starch content. The reaction mixture was then cooled to 65°C and treated with cellulose- and β-glucan hydrolysing enzymes at 65-55°C (GC440, Genencor and Ultraflo L, Novozymes, both 2 % of the starting material) at neutral pH for 2 hours, after which the precipitate was separated by a separator and spray dried. The composition contained 46 % protein, 12 % lipids in total, including >3 % emulsifying lipids.

### Example 6

An oat bran rich fraction was used as a starting material for the preparation of a composition containing protein hydrolysates and emulsifying lipids. The starting material contained 17 % protein, 8 % lipids (including >2 % emulsifying lipids), 17 % fibre and 46 % other carbohydrates.

The oat bran rich fraction was mixed with water (1:10) and heated to 90°C to gelatinize the starch. A thermostable α-amylase (Spezyme FRED, Genencor, 1 % of the starting material) was added into the reaction vessel and allowed to react for 2 hours at 90 °C to reduce the starch content. The reaction mixture was treated with cellulose- and β-glucan hydrolysing enzymes at 65-55°C (GC440, Genencor and Ultraflo L, Novozymes, both 2 % of the starting material) at neutral pH for 5 hours to hydrolyse the fibre components. The fibre content of the oat bran rich fraction was higher than that of the fractions used in other examples and thus also extended hydrolysis time with cellulase and β-glucanase was used. The resulting mixture was then hydrolysed at 37°C with pepsin (Sigma, pH 2, 8 hours, 1 % of the protein content of the starting material). The reaction mixture was neutralised and heated to 90°C for 30 min to inactivate the enzyme. The precipitate was separated by centrifugation and spray dried.

### Example 7

### In vitro activity test

The compositions from examples 1 and 2 were compared to a commercial cholesterol lowering agent, soy protein hydrolysate - phospholipid complex CSPHP (Kyowa Hakko). The commercial cholesterol lowering agent CSPHP is known to bind bile acid taurocholate *in vitro* and to increase fecal bile acid excretion in rats. This agent lowers serum total and LDL cholesterol in humans and in rats. Bile acid binding and increased fecal bile acid excretion is known to be one of the mechanisms that many cholesterol lowering ingredients utilise.

The bile acid binding capacity of the compositions from examples 1 and 2 was compared to the bile acid binding capacity of CSPHP in an *in vitro* test. From 100 mg to 1000 mg test material (CSPHP, the composition from example 1 or the composition from example 2) was weighed and mixed with a known amount of cholic acid (about 10 mM) in phosphate buffer (pH 7.4) and shaken overnight. The supernatant was separated by centrifugation. The cholic acid concentration in the supernatant (unbound cholic acid) was measured by gas chromatography with an external standard method. The amount of the cholic acid bound to the test material was calculated from the amount of the unbound cholic acid and the amount of the total cholic acid used in the test. The amount of the bound cholic acid (mg) was calculated per g test material as a mean value of two duplicate analyses.

**Table 1. In vitro activity test results**

| | Bound cholic acid mg/ g test material |
|---|---|
| Composition from example 1 | 21,8 |
| Composition from example 2 | 24,4 |
| Commercial agent CSPHP | 19,6 |

The results shown in Table 1 indicate that the compositions of the present invention worked in a similar or even in a better way in the test than a commercial cholesterol lowering agent CSPHP that is known to lower serum total and LDL cholesterol levels. The oat based compositions of the present invention bound 11-25 % more cholic acid than the commercial cholesterol lowering agent, which indicates the potential of the oat based compositions of the present invention as serum lipid profile improving ingredients.

### Example 8

### Animal test

The lipid profile improving effect of the compositions containing oat fraction comprising protein and emulsifying lipids (composition from example 5 = composition C) and oat fraction comprising oat protein hydrolysate and emulsifying lipids (composition from example 2 = composition B) was studied by using LDL-receptor deficient female mice as test animals.

The aim of the test was to study the serum total cholesterol and triglyceride lowering effect that could be obtained by using the compositions of the present invention as part of an atherogenic diet (contained 0.25 % by weight cholesterol and 18 % by weight of cocoa butter).

The animals were assigned into 3 groups (n=8 in each group) and fed experimental diets for 4 weeks, whereafter the changes in serum cholesterol and triglycerides was measured.

As basic diet Clinton/Cybulsky (D12106p, without protein), from Research Diets Inc. was used.

All the experimental diets were formulated to contain 20 % protein as N equivalents. In the control group (group A) the sole protein source was casein (88 % purity). In the test groups (B and C) the protein source was half casein and half (as N equivalents) composition B or composition C. Otherwise the diets had the same composition.

**Table 2. Experimental results**

| | **Group A control** | **Group B composition B (Example 2)** | **Group C composition C (Example 5)** |
|---|---|---|---|
| Serum total cholesterol (mmol/l), mean | 18.7 | 14.6 | 16.8 |
| Change (%) compared to control group | | -22.1 | -10.4 |
| Serum triglycerides (mmol/l), mean | 3.7 | 2.7 | 3.0 |
| Change (%) compared to control group | | -26.2 | -18.5 |

As shown in Table 2, both compositions from example 2 (group B) and from example 5 (group C) had serum cholesterol lowering and serum triglyceride lowering effect compared to the control group containing casein as a sole protein source. The results were surprising, especially as half of the N-source fed was inactive casein. Especially serum triglyserides were lowered surprisingly well in groups B and C.

### Example 9

### Yogurt-like cereal product

Oat composition from example 1 was used in a preparation of fermented, yogurt-like cereal product.

| | |
|---|---|
| Oat milk | 72.0 % |
| Strawberry jam | 20.0 % |
| Composition from example 1 | 5.0 % |
| Oat bran | 2.5 % |
| Pectin | 0.5 % |

The yoghurt was prepared by conventional yoghurt preparation methods and fermented with *Bifidobacteria* culture. A 150 g serving of the product contained 7.5 g of the composition from the example 1.

### Example 10

### Food bar

Composition from example 3 was used for preparation of food bars.

| | |
|---|---|
| oat flakes | 32.0 % |
| oat bran | 8.0 % |
| corn syrup | 15.0 % |
| brown sugar | 5.0 % |
| concentrated apple juice | 5.5 % |
| apple | 2.0 % |
| raisin | 3.0 % |
| vegetable fat | 12.0 % |
| salt | 0.5 % |
| flavouring | 0.5 % |
| composition from example 3 | 16 % |
| lecithin | 0.5 % |

A 45 g food bar contained 7.2 g of the composition from example 3.

### Example 11

### Yoghurt-like product

| | |
|---|---|
| Soy milk | 75.5 % |
| Strawberry jam | 20.0 % |
| Composition from example 4 | 4.0 % |
| Pectin | 0.5 % |

The yoghurt was prepared by conventional soy yoghurt preparation methods and fermented with *Bifidobacteria* culture. A 150 g serving of the product contained 6.0 g of the composition from the example 4.

### Example 12

### Flour mix for preparation of cookies

| | |
|---|---|
| Sugar | 19.0% |
| Wheat flour | 37.5 % |
| Composition from example 6 | 15.3 % |
| Salt | 0.5 % |
| Vanilla flavour | 0.2 % |
| Sodium bicarbonate | 0.5 % |
| Chocolate chips | 27.0 % |

Shortening (34 % of the flour mix), eggs (10 % of the flour mix) and water (16 % of the flour mix) are added into the flour mix when the cookies are prepared. The cookies are baked at 180°C for 8-10 min. A serving of two cookies (24 g) contains 2.4 g composition from the example 6.

### Example 13

### Wheat pasta

| | |
|---|---|
| Soft wheat flour | 45.0 % |
| Durum wheat flour | 43.0 % |
| Composition from example 2 | 12.0 % |

The pasta was produced using a conventional high-temperature pasta process (Pavan Mapimpianti/Italy; Termo-Active-System). A serving of 70 g contained 8.4 g composition form the example 2.

### Example 14

### Meat balls

| | |
|---|---|
| Minced meat, 17 % fat | 57.4 % |
| Water | 14.2 % |
| Eggs | 13.5 % |
| Onion (chopped) | 7.0 % |
| Composition from example 1 | 7.0 % |
| Salt | 0.9 % |

The meat balls were prepared in oven at 200 °C. A serving of 120 g contained 12.4 g of the composition from example 1.

### Example 15

### Vegetable soup powder

| | |
|---|---|
| Dehydrated vegetables (tomatoes, onions, garlic) | 33.0 % |
| Potato starch | 18.0 % |
| Maltodextrin | 4.3 % |
| Composition from example 5 | 28.0 % |
| Partially hydrogenated soybean oil | 6.0 % |
| Sucrose | 7.0 % |
| Salt | 3.0 % |
| Guar gum | 0.2 % |
| Spices and flavours | 0.5 % |

A serving of 25 g contained 7 g of the composition from example 5.

### Example 16

### Yogurt-like cereal product

Oat composition from example 1 and plant stanol ester was used in a preparation of fermented, yogurt-like cereal product.

| | |
|---|---|
| Oat milk | 70.0 % |
| Strawberry jam | 20.0 % |
| Composition from example 1 | 5.0 % |
| Oat bran | 2.5 % |
| Pectin | 0.5 % |
| Plant stanol ester | 2.0 % |

The yoghurt was prepared by conventional yoghurt preparation methods. A 150 g serving of the product contained 7.5 g of the composition from the example 1 and 3 g plant stanol ester (1.8 g as sterol equivalents).

## Claims

1. A therapeutical composition for oral use comprising at least 25 % by dry weight protein and at least 1 % by dry weight emulsifying lipids, both the protein and the emulsifying lipids being obtained from the same oat fraction, and the weight ratio of protein to emulsifying lipids is from 1:0.01 to 1:0.6, said composition further comprising β-glucan in an amount of at most 8 % by dry weight, and wherein the emulsifying lipids comprise phospholipids and glycolipids and the weight ratio of the phospholipids to the glycolipids is from 1:0.7 to 1:6.

2. The therapeutical composition according to claim 1, wherein the composition is obtainable from an oat endosperm-rich fraction comprising at least 10 % by dry weight protein and at least 1 % by dry weight emulsifying lipids.

3. The therapeutical composition according to claim 1, wherein the composition is obtainable from a whole grain based oat fraction comprising at least 10 % by dry weight protein and at least 1 % by dry weight emulsifying lipids.

4. The therapeutical composition according to claim 1, wherein the composition is obtainable from an oat bran-rich fraction comprising at least 10 % by dry weight protein and at least 1 % by dry weight emulsifying lipids.

5. The therapeutical composition according to any one of claims 1 to 4, wherein the composition comprises at least 30 %, more preferably at least 35 %, and most preferably at least 40 % by dry weight protein.

6. The therapeutical composition according to any one of claims 1 to 5, wherein the composition comprises at least 2 %, more preferably at least 3 %, still more preferably at least 4 %, even more preferably at least 5 % and most preferably at least 6 % by dry weight emulsifying lipids.

7. The therapeutical composition according to any one of claims 1 to 6, wherein the composition comprises from 25 to 99 %, preferably from 30 to 98 %, more preferably from 35 to 97.5 %, still more preferably from 40 to 97 % and most preferably from 40 to 94 % by dry weight protein.

8. The therapeutical composition according to claim 7, wherein the composition comprises from 1 to 15 %, preferably from 2 to 15 %, more preferably from 2.5 to 15 %, still more preferably from 3 to 15 %, even more preferably from 4 to 15 %, still even more preferably from 5 to 15 %, and most preferably from 6 to 15 % by dry weight emulsifying lipids.

9. The therapeutical composition according to any one of claims 1 to 6, wherein the composition comprises from 25 to 98 %, preferably from 30 to 98 %, more preferably from 35 to 97.5 %, still more preferably from 40 to 97 % and most preferably from 40 to 94 % by dry weight protein.

10. The therapeutical composition according to claim 9, wherein the composition comprises from 2 to 15 %, preferably from 2 to 10 %, more preferably from 2.5 to 10 %, still more preferably from 3 to 8 %, even more preferably from 4 to 8 %, still even more preferably from 5 to 8 % and most preferably from 6 to 8 % by dry weight emulsifying lipids.

11. The therapeutical composition according to any one of claims 1 to 10 further comprising dietary fibre in an amount of at most 15 %, preferably at most 10 %, more preferably at most 5 % and most preferably at most 3 % by dry weight.

12. The therapeutical composition according to any one of claims 1 to 11 comprising β-glucan in an amount of at most 5 %, preferably at most 3 % and more preferably at most 2 % by dry weight.

13. The therapeutical composition according to any of claims 1 to 12, wherein the weight ratio of the phospholipids to the glycolipids is from 1:1 to 1:5, preferably from 1:1 to 1:3.

14. The therapeutical composition according to any one of claims 1 to 13 further comprising plant sterol.

15. The therapeutical composition according to claim 14, wherein the weight ratio of plant sterol to protein is from 1:0.02 to 1:150, preferably from 1:0.2 to 1:30, more preferably from 1:0.4 to 1:12.5 and most preferably from 1:1 to 1:5.

16. A therapeutical composition for oral use comprising at least 15 % by dry weight protein hydrolysate and at least 1 % by dry weight emulsifying lipids, both the protein and the emulsifying lipids being obtained from the same oat fraction which has been subjected to hydrolysis, and the weight ratio of protein hydrolysate to the emulsifying lipids is from 1:0.01 to 1:1, said composition further comprising β-glucan in an amount of at most 8 % by dry weight, and wherein the emulsifying lipids comprise phospholipids and glycolipids and the weight ratio of the phospholipids to the glycolipids is from 1:0.7 to 1:6.

17. The therapeutical composition according to claim 16, wherein the composition is obtainable by hydrolysing an oat endosperm-rich fraction comprising at least 10 % by dry weight protein and at least 1 % by dry weight emulsifying lipids.

18. The therapeutical composition according to claim 16, wherein the composition is obtainable by hydrolysing a whole grain based oat fraction comprising at least 10 % by dry weight protein and at least 1 % by dry weight emulsifying lipids.

19. The therapeutical composition according to claim 16, wherein the composition is obtainable by hydrolysing an oat bran-rich fraction comprising at least 10 % by dry weight protein and at least 1 % by dry weight emulsifying lipids.

20. The therapeutical composition according to any one of claim 16 to 19, wherein the composition comprises at least 20 %, preferably at least 30 % by dry weight protein hydrolysate.

21. The therapeutical composition according to any one of claims 16 to 20, wherein the composition comprises at least 2 %, preferably at least 3 %, more preferably at least 4 %, still more preferably at least 5 % and most preferably at least 6 % by dry weight emulsifying lipids.

22. The therapeutical composition according to any one of claims 16 to 21, wherein the weight ratio of the phospholipids to the glycolipids is from 1:1 to 1:5, preferably from 1:1 to 1:3.

23. The therapeutical composition according to any one of claims 16 to 22, wherein the composition further comprises dietary fibre in an amount of at most 15 %, preferably at most 10 %, more preferably at most 5 % and most preferably at most 3 % by dry weight.

24. The therapeutical composition according to any one of claims 16 to 23 comprising β-glucan in an amount of at most 5 %, preferably at most 3 % and more preferably at most 2 % by dry weight.

25. The therapeutical composition according to any one of claims 16 to 24, wherein the amount of protein hydrolysate is from 15 to 99 %, preferably from 20 to 98 %, more preferably from 30 to 97 % and most preferably from 30 to 94 % by dry weight.

26. The therapeutical composition according to claim 25, wherein the amount of emulsifying lipids is from 1 to 15 %, preferably from 2 to 15 %, more preferably from 2.5 to 15 %, still more preferably from 3 to 15 %, even more preferably from 4 to 15 %, still even more preferably from 5 to 15 %, and most preferably from 6 to 15 % by dry weight.

27. The therapeutical composition according to any one of claims 16 to 24, wherein the amount of protein hydrolysate is from 15 to 98 %, preferably from 20 to 98 %, more preferably from 30 to 97 % and most preferably from 30 to 94 % by dry weight.

28. The therapeutical composition according to claim 27, wherein the amount of emulsifying lipids is from 2 to 15 %, preferably from 2 to 10 % by dry weight, more preferably from 2.5 to 10 %, still more preferably from 3 to 8 %, even more preferably from 4 to 8 %, still even more preferably from 5 to 8 % and most preferably from 6 to 8 % by dry weight.

29. The therapeutical composition according to any one of claims 16 to 28, wherein the protein hydrolysate comprises resistant protein.

30. The therapeutical composition according to any one of claims 16 to 29, wherein the protein hydrolysate has a molecular weight of 300-100 000 D, preferably 500-50 000 D, more preferably 500-30 000 D, even more preferably 900-30 000 D and most preferably 1 600-30 000 D.

31. The therapeutical composition according to any one of claims 16 to 30, wherein the degree of hydrolysis of the protein is from 1 to 40 %, preferably from 3 to 30 % and most preferably from 5 to 20 %.

32. The therapeutical composition according to any one of claims 16 to 31 further comprising plant sterol.

33. The therapeutical composition according to claim 32, wherein the weight ratio of plant sterol to protein is from 1:0.02 to 1:150, preferably from 1:0.2 to 1:30, more preferably from 1:0.4 to 1:12.5 and most preferably from 1:1 to 1:5.

34. A food product comprising at least one basic nutritional ingredient and a therapeutical composition according to any one of claims 1 to 33.

35. Use of a composition comprising according to any one of claims 1 to 33 for the manufacture of a pharmaceutical or nutraceutical or food product for improving the serum lipid profile.

36. A method for preparing a therapeutical composition according to any one of claims 1 to 15 said method comprising treating an oat fraction comprising both protein and emulsifying lipids and carbohydrates to remove carbohydrates, preferably by enzymatic hydrolysis, and to obtain said therapeutic composition having an increased content of protein.

37. A method for preparing a therapeutical composition according to any one of claims 16 to 33 said method comprising hydrolysing an oat fraction comprising both protein and emulsifying lipids to obtain said therapeutical composition.

38. The method according to claim 37 wherein the degree of hydrolysis is from 1 to 40 %, preferably from 3 to 30 % and most preferably from 5 to 20 %.

39. The method according to claim 37 or 38 wherein the oat fraction is hydrolysed by enzymatic hydrolysis, preferably by using a protease.

## Patentansprüche

1. Therapeutische Zusammensetzung zur oralen Verwendung, enthaltend mindestens 25 % bezogen auf das Trockengewicht Protein und mindestens 1 % bezogen auf das Trockengewicht emulgierende Lipide, wobei sowohl das Protein als auch die emulgierenden Lipide erhalten sind aus der gleichen Hafer-Fraktion und das Gewichtsverhältnis von Protein zu emulgierenden Lipiden 1 : 0,01 bis 1 : 0,6 beträgt, und worin die Zusammensetzung außerdem β-Glukan in einer Menge von maximal 8 % bezogen auf das Trockengewicht enthält und worin die emulgierenden Lipide Phospholipide und Glykolipide enthalten und worin das Gewichtsverhältnis der Phospholipide zu den Glykolipiden 1 : 0,7 bis 1 : 6 beträgt.

2. Therapeutische Zusammensetzung gemäß Anspruch 1, worin die Zusammensetzung erhältlich ist aus einer Endosperm-reichen Hafer-Fraktion, enthaltend mindestens 10 % bezogen auf das Trockengewicht Protein und mindestens 1 % bezogen auf das Trockengewicht emulgierende Lipide.

3. Therapeutische Zusammensetzung gemäß Anspruch 1, worin die Zusammensetzung erhältlich ist aus einer Vollkorn-basierten Hafer-Fraktion, enthaltend mindestens 10 % bezogen auf das Trockengewicht Protein und mindestens 1 % bezogen auf das Trockengewicht emulgierende Lipide.

4. Therapeutische Zusammensetzung gemäß Anspruch 1, worin die Zusammensetzung erhältlich ist aus einer Haferkleie-reichen Fraktion, enthaltend mindestens 1 % bezogen auf das Trockengewicht Protein und mindestens 1 % bezogen auf das Trockengewicht emulgierende Lipide.

5. Therapeutische Zusammensetzung gemäß einem der Ansprüche 1 bis 4, worin die Zusammensetzung mindestens 30 %, bevorzugter mindestens 35 %, am meisten bevorzugt mindestens 40 % bezogen auf das Trockengewicht Protein enthält.

6. Therapeutische Zusammensetzung gemäß einem der Ansprüche 1 bis 5, worin die Zusammensetzung mindestens 2 %, bevorzugter mindestens 3 %, noch bevorzugter mindestens 4 %, noch bevorzugter mindestens 5 % und am meisten bevorzugt mindestens 6 % bezogen auf das Trockengewicht emulgierende Lipide enthält.

7. Therapeutische Zusammensetzung gemäß einem der Ansprüche 1 bis 6, worin die Zusammensetzung 25 bis 99 %, bevorzugt 30 bis 98 %, bevorzugter 35 bis 97,5 %, noch bevorzugter 40 bis 97 % und am meisten bevorzugt 40 bis 94 % bezogen auf das Trockengewicht Protein enthält.

8. Therapeutische Zusammensetzung gemäß Anspruch 7, worin die Zusammensetzung 1 bis 15 %, bevorzugt 2 bis 15 %, bevorzugter 2,5 bis 15 %, noch bevorzugter 3 bis 15 %, noch bevorzugter 4 bis 15 %, noch bevorzugter 5 bis 15 % und am meisten bevorzugt 6 bis 15 % bezogen auf das Trockengewicht emulgierende Lipide umfasst.

9. Therapeutische Zusammensetzung gemäß einem der Ansprüche 1 bis 6, worin die Zusammensetzung 25 bis 98 %, bevorzugt 30 bis 98 %, bevorzugter 35 bis 97,5 %, noch bevorzugter 40 bis 97 % und am meisten bevorzugt 40 bis 94 % bezogen auf das Trockengewicht Protein enthält.

10. Therapeutische Zusammensetzung gemäß Anspruch 9, worin die Zusammensetzung 2 bis 15 %, bevorzugt 2 bis 10 %, bevorzugter 2,5 bis 10 %, noch bevorzugter 3 bis 8 %, noch bevorzugter 4 bis 8 %, noch bevorzugter 5 bis 8 % und am meisten bevorzugt 6 bis 8 % bezogen auf das Trockengewicht emulgierende Lipide enthält.

11. Therapeutische Zusammensetzung gemäß einem der Ansprüche 1 bis 10, die außerdem diätetische Fasern in einer Menge von maximal 15 %, bevorzugt maximal 10 %, bevorzugter maximal 5 % und am meisten bevorzugt maximal 3 % bezogen auf das Trockengewicht enthält.

12. Therapeutische Zusammensetzung gemäß einem der Ansprüche 1 bis 11, enthaltend β-Glukan in einer Menge von maximal 5 %, bevorzugt maximal 3 % und bevorzugter maximal 2 % bezogen auf das Trockengewicht enthält.

13. Therapeutische Zusammensetzung gemäß einem der Ansprüche 1 bis 12, worin das Gewichtsverhältnis der Phospholipide zu den Glykolipiden 1 : 1 bis 1 : 5, bevorzugt 1 : 1 bis 1 : 3 beträgt.

14. Therapeutische Zusammensetzung gemäß einem der Ansprüche 1 bis 13, enthaltend außerdem Pflanzensterin.

15. Therapeutische Zusammensetzung gemäß Anspruch 14, worin das Gewichtsverhältnis von Pflanzensterin zu Protein 1 : 0,02 bis 1 : 150, bevorzugt 1 : 0,2 bis 1 : 30, bevorzugter 1 : 0,4 bis 1 : 12,5 und am meisten bevorzugt von 1 : 1 bis 1 : 5 beträgt.

16. Therapeutische Zusammensetzung zur oralen Verwendung, enthaltend mindestens 15 % bezogen auf das Trockengewicht Proteinhydrolysat und mindestens 1 % bezogen auf das Trockengewicht emulgierende Lipide, wobei sowohl das Protein als auch die emulgierenden Lipide erhalten sind aus der gleichen Hafer-Fraktion, welche einer Hydrolyse unterworfen wurde und worin das Gewichtsverhältnis von Proteinhydrolysat zu emulgierenden Lipiden 1 : 0,01 bis 1 : 1 beträgt, und worin die Zusammensetzung außerdem β-Glukan in einer Menge von maximal 8 % bezogen auf das Trockengewicht enthält und worin die emulgierenden Lipide Phospholipide und Glykolipide enthalten und das Gewichtsverhältnis der PHospholipide zu den Glykolipiden 1 : 0,7 bis 1 : 6 beträgt.

17. Therapeutische Zusammensetzung gemäß Anspruch 16, worin die Zusammensetzung erhältlich ist durch Hydrolyse einer Endosperm-reichen Hafer-Fraktion, enthaltend mindestens 10 % bezogen auf das Trockengewicht Protein und mindestens 1 % bezogen auf das Trockengewicht emulgierende Lipide.

18. Therapeutische Zusammensetzung gemäß Anspruch 16, worin die Zusammensetzung erhältlich ist durch Hydrolyse einer Vollkorn-basierten Hafer-Fraktion, enthaltend mindestens 10 % bezogen auf das Trockengewicht Protein und mindestens 1 % bezogen auf das Trockengewicht emulgierender Lipide.

19. Therapeutische Zusammensetzung gemäß Anspruch 16, worin die Zusammensetzung erhältlich ist durch Hydrolyse einer Haferkleie-reichen Fraktion, enthaltend mindestens 10 % bezogen auf das Trockengewicht Protein und mindestens 1 % bezogen auf das Trockengewicht emulgierender Lipide.

20. Therapeutische Zusammensetzung gemäß einem der Ansprüche 16 bis 19, worin die Zusammensetzung mindestens 20 %, bevorzugt mindestens 30 % bezogen auf das Trockengewicht Proteinhydrolysat enthält.

21. Therapeutische Zusammensetzung gemäß einem der Ansprüche 16 bis 20, worin die Zusammensetzung mindestens 2 %, bevorzugt mindestens 3 %, bevorzugter mindestens 4 %, noch bevorzugter mindestens 5 % und am meisten bevorzugt mindestens 6 % bezogen auf das Trockengewicht emulgierende Lipide umfasst.

22. Therapeutische Zusammensetzung gemäß einem der Ansprüche 16 bis 21, worin das Gewichtsverhältnis der Phospholipide zu den Glykolipiden 1 : 1 bis 1 : 5, bevorzugt 1 : 1 bis 1 : 3 beträgt.

23. Therapeutische Zusammensetzung gemäß einem der Ansprüche 16 bis 22, worin die Zusammensetzung außerdem diätetische Fasern in einer Menge von maximal 15 %, bevorzugt maximal 10 %, bevorzugter maximal 5 % und am meisten bevorzugt maximal 3 % bezogen auf das Trockengewicht enthält.

24. Therapeutische Zusammensetzung gemäß einem der Ansprüche 16 bis 23, enthaltend β-Glukan in einer Menge von maximal 5 %, bevorzugt maximal 3 % und am meisten bevorzugt maximal 2 % bezogen auf das Trockengewicht.

25. Therapeutische Zusammensetzung gemäß einem der Ansprüche 16 bis 24, worin der Gehalt von Proteinhydrolysat 15 bis 99 %, bevorzugt 20 bis 98 %, bevorzugter 30 bis 97 % und am meisten bevorzugt 30 bis 94 % bezogen auf das Trockengewicht beträgt.

26. Therapeutische Zusammensetzung gemäß Anspruch 25, worin der Gehalt emulgierender Lipide 1 bis 15 %, bevorzugt 2 bis 15 %, bevorzugter 2,5 bis 15 %, noch bevorzugter 3 bis 15 %, noch bevorzugter 4 bis 15 %, noch bevorzugter 5 bis 15 % und am meisten bevorzugt 6 bis 15 % bezogen auf das Trockengewicht beträgt.

27. Therapeutische Zusammensetzung gemäß einem der Ansprüche 16 bis 24, worin der Gehalt an Proteinhydrolysat 15 bis 98 %, bevorzugt 20 bis 98 %, bevorzugter 30 bis 97 % und am meisten bevorzugt 30 bis 94 % bezogen auf das Trockengewicht beträgt.

28. Therapeutische Zusammensetzung gemäß Anspruch 27, worin der Gehalt emulgierender Lipide 2 bis 15 %, bevorzugt 2 bis 10 % bezogen auf das Trockengewicht, bevorzugter 2,5 bis 10 %, noch bevorzugter 3 bis 8 %, noch bevorzugter 4 bis 8 %, noch bevorzugter 5 bis 8 % und am meisten bevorzugt 6 bis 8 % bezogen auf das Trockengewicht beträgt.

29. Therapeutische Zusammensetzung gemäß einem der Ansprüche 16 bis 28, worin das Proteinhydrolysat resistentes Protein enthält.

30. Therapeutische Zusammensetzung gemäß einem der Ansprüche 16 bis 29, worin das Proteinhydrolysat ein Molekulargewicht von 300 bis 100000 D, bevorzugt 500 bis 50000 D, bevorzugter 500 bis 30000 D, noch bevorzugter 900 bis 30000 D und am meisten bevorzugt 1600 bis 30000 D aufweist.

31. Therapeutische Zusammensetzung gemäß einem der Ansprüche 16 bis 30, worin der Grad der Hydrolyse des Proteins 1 bis 40 %, bevorzugt 3 bis 30 % und am meisten bevorzugt 5 bis 20 % beträgt.

32. Therapeutische Zusammensetzung gemäß einem der Ansprüche 16 bis 31, welches außerdem Pflanzensterin enthält.

33. Therapeutische Zusammensetzung gemäß Anspruch 32, worin das Gewichtsverhältnis von Pflanzensterin zu Protein 1 : 0,02 bis 1 : 150, bevorzugt 1 : 0,2 bis 1 : 30, noch bevorzugter 1 : 0,4 bis 1 : 12,5 und am meisten bevorzugt 1 : 1 bis 1 : 5 beträgt.

34. Nahrungsmittelprodukt, enthaltend mindestens einen basischen Nährbestandteil und eine therapeutische Zusammensetzung gemäß einem der Ansprüche 1 bis 33.

35. Verwendung einer Zusammensetzung gemäß einem der Ansprüche 1 bis 33 zur Herstellung eines pharmazeutischen oder nutrazeutischen Nahrungsmittelprodukts zur Verbesserung des Serum-Lipid-Profils.

36. Verfahren zur Herstellung einer therapeutischen Zusammensetzung gemäß einem der Ansprüche 1 bis 15, worin das Verfahren die Behandlung einer Hafer-Fraktion, enthaltend Protein und emulgierende Lipide und Kohlenhydrate, zur Entfernung der Kohlenhydrate, bevorzugt durch enzymatische Hydrolyse, umfasst, um die therapeutische Zusammensetzung mit erhöhtem Proteingehalt zu erhalten.

37. Verfahren zur Herstellung einer therapeutischen Zusammensetzung gemäß einem der Ansprüche 16 bis 33, worin das Verfahren die Hydrolyse einer Hafer-Fraktion, enthaltend Protein und emulgierende Lipide umfasst, um die pharmazeutische Zusammensetzung zu erhalten.

38. Verfahren gemäß Anspruch 37, worin der Grad der Hydrolyse 1 bis 40 %, bevorzugt 3 bis 30 % und am meisten bevorzugt 5 bis 20 % beträgt.

39. Verfahren gemäß Anspruch 37 oder 38, worin die Hafer-Fraktion durch enzymatische Hydrolyse hydrolysiert wird, bevorzugt unter Verwendung einer Protease.

## Revendications

1. Une composition thérapeutique pour une utilisation orale comprenant au moins 25% en poids sec de protéine et au moins 1% en poids sec de lipides émulsifiants, à la fois la protéine et les lipides émulsifiants étant obtenus à partir de la même fraction d'avoine, et le rapport pondéral protéine/lipides émulsifiants étant de 1:0,01 à 1:0,6, ladite composition comprenant en outre du β-glucane en une quantité d'au plus 8% en poids sec, et dans laquelle les lipides émulsifiants comprennent des phospholipides et des glycolipides, et le rapport pondéral phospholipides:glycolipides est de 1:0,7 à 1:6.

2. La composition thérapeutique selon la revendication 1, dans laquelle la composition peut être obtenue à partir d'une fraction d'avoine riche en endosperme comprenant au moins 10% en poids sec de protéine et au moins 1% en poids sec de lipides émulsifiants.

3. La composition thérapeutique selon la revendication 1, dans laquelle la composition peut être obtenue à partir d'une fraction d'avoine à base de grains entiers comprenant au moins 10% en poids sec de protéine et au moins 1% en poids sec de lipides émulsifiants.

4. La composition thérapeutique selon la revendication 1, dans laquelle la composition peut être obtenue à partir d'une fraction riche en son d'avoine comprenant au moins 10% en poids sec de protéine et au moins 1% en poids sec de lipides émulsifiants.

5. La composition thérapeutique selon l'une quelconque des revendications 1 à 4, dans laquelle la composition comprend au moins 30%, plus préférentiellement au moins 35%, et de manière préférée au moins 40% en poids sec de protéine.

6. La composition thérapeutique selon l'une quelconque des revendications 1 à 5, dans laquelle la composition comprend au moins 2%, plus préférentiellement au moins 3%, encore plus préférentiellement au moins 4%, encore plus préférentiellement au moins 5% et de manière préférée au moins 6 % en poids sec de lipides émulsifiants.

7. La composition thérapeutique selon l'une quelconque des revendications 1 à 6, dans laquelle la composition comprend de 25 à 99%, de préférence de 30 à 98%, plus préférentiellement de 35 à 97,5%, encore plus préférentiellement de 40 à 97% et de manière préférée de 40 à 94% en poids sec de protéine.

8. La composition thérapeutique selon la revendication 7, dans laquelle la composition comprend de 1 à 15%, de préférence de 2 à 15%, plus préférentiellement de 2,5 à 15%, encore plus préférentiellement de 3 à 15%, encore plus préférentiellement de 4 à 15%, encore plus préférentiellement de 5 à 15%, et de manière préférée de 6 à 15% en poids sec de lipides émulsifiants.

9. La composition thérapeutique selon l'une quelconque des revendications 1 à 6, dans laquelle la composition comprend de 25 à 98%, de préférence de 30 à 98%, plus préférentiellement de 35 à 97,5%, encore plus préférentiellement de 40 à 97% et de manière préférée de 40 à 94% en poids sec de protéine.

10. La composition thérapeutique selon la revendication 9, dans laquelle la composition comprend de 2 à 15%, de préférence de 2 à 10%, plus préférentiellement de 2,5 à 10%, encore plus préférentiellement de 3 à 8%, encore plus préférentiellement de 4 à 8% , encore plus préférentiellement de 5 à 8% et de manière préférée de 6 à 8% en poids sec de lipides émulsifiants.

11. La composition thérapeutique selon l'une quelconque des revendications 1 à 10, comprenant en outre des fibres alimentaires en une quantité d'au plus 15%, de préférence d'au plus 10%, plus préférentiellement d'au plus 5% et de manière préférée d'au plus 3% en poids sec.

12. La composition thérapeutique selon l'une quelconque des revendications 1 à 11, comprenant du β-glucane en une quantité d'au plus 5%, de préférence d'au plus 3%, et de manière préférée d'au plus 2% en poids sec.

13. La composition thérapeutique selon l'une quelconque des revendications 1 à 12, dans laquelle le rapport pondéral phospholipides:glycolipides est de 1:1 à 1:5, de préférence de 1:1 à 1:3.

14. La composition thérapeutique selon l'une quelconque des revendications 1 à 13, comprenant en outre du stérol végétal.

15. La composition thérapeutique selon la revendication 14, dans laquelle le rapport pondéral stérol végétal:protéine est de 1:0,02 à 1:150, de préférence de 1:0,2 à 1:30, plus préférentiellement de 1:0,4 à 1:12,5 et de manière préférée de 1:1 à 1:5.

16. Une composition thérapeutique pour une utilisation orale comprenant au moins 15% en poids sec d'hydrolysat de protéine et au moins 1% en poids sec de lipides émulsifiants, à la fois la protéine et les lipides émulsifiants étant obtenus à partir de la même fraction d'avoine qui a été soumise à l'hydrolyse, et le rapport pondéral hydrolysat de protéine:lipides émulsifiants étant de 1:0,01 à 1:1, ladite composition comprenant en outre du β-glucane en une quantité d'au plus 8% en poids sec, et dans laquelle les lipides émulsifiants comprennent des phospholipides et des glycolipides, et le rapport pondéral phospholipides:glycolipides est de 1:0,7 à 1:6.

17. La composition thérapeutique selon la revendication 16, dans laquelle la composition peut être obtenue par hydrolyse d'une fraction d'avoine riche en endosperme comprenant au moins 10% en poids sec de protéine et au moins 1% en poids sec de lipides émulsifiants.

18. La composition thérapeutique selon la revendication 16, dans laquelle la composition peut être obtenue par hydrolyse d'une fraction d'avoine à base de grains entiers comprenant au moins 10% en poids sec de protéine et au moins 1% en poids sec de lipides émulsifiants.

19. La composition thérapeutique selon la revendication 16, dans laquelle la composition peut être obtenue par hydrolyse d'une fraction riche en son d'avoine comprenant au moins 10% en poids sec de protéine et au moins 1% en poids sec de lipides émulsifiants.

20. La composition thérapeutique selon l'une quelconque des revendications 16 à 19, dans laquelle la composition comprend au moins 20%, de préférence au moins 30% en poids sec d'hydrolysat de protéine.

21. La composition thérapeutique selon l'une quelconque des revendications 16 à 20, dans laquelle la composition comprend au moins 2%, de préférence au moins 3%, plus préférentiellement au moins 4%, encore plus préférentiellement au moins 5% et de manière préférée au moins 6% en poids sec de lipides émulsifiants.

22. La composition thérapeutique selon l'une quelconque des revendications 16 à 21, dans laquelle le rapport pondéral phospholipides:glycolipides est de 1:1 à 1:5, de préférence de 1:1 à 1:3.

23. La composition thérapeutique selon l'une quelconque des revendications 16 à 22, dans laquelle la composition comprend en outre des fibres alimentaires en une quantité d'au plus 15%, de préférence d'au plus 10%, plus préférentiellement d'au plus 5% et de manière préférée d'au plus 3% en poids sec.

24. La composition thérapeutique selon l'une quelconque des revendications 16 à 23, comprenant du β-glucane en une quantité d'au plus 5%, de préférence d'au plus 3%, et plus préférentiellement d'au plus 2% en poids sec.

25. La composition thérapeutique selon l'une quelconque des revendications 16 à 24, dans laquelle la quantité d'hydrolysat de protéine est de 15 à 99%, de préférence de 20 à 98%, plus préférablement de 30 à 97% et de manière préférée de 30 à 94% en poids sec.

26. La composition thérapeutique selon la revendication 25, dans laquelle la quantité de lipides émulsifiants est de 1 à 15%, de préférence de 2 à 15%, plus préférentiellement de 2,5 à 15%, encore plus préférentiellement de 3 à 15%, encore plus préférentiellement de 4 à 15%, encore plus préférentiellement de 5 à 15%, et de manière préférée de 6 à 15% en poids sec.

27. La composition thérapeutique selon l'une quelconque des revendications 16 à 24, dans laquelle la quantité d'hydrolysat de protéine est de 15 à 98%, de préférence de 20 à 98%, plus préférentiellement de 30 à 97% et de manière préférée de 30 à 94% en poids sec.

28. La composition thérapeutique selon la revendication 27, dans laquelle la quantité de lipides émulsifiants est de 2 à 15%, de préférence de 2 à 10% en poids sec, plus préférentiellement de 2,5 à 10%, encore plus préférentiellement de 3 à 8%, encore plus préférentiellement de 4 à 8%, encore plus préférentiellement de 5 à 8% et de manière préférée de 6 à 8% en poids sec.

29. La composition thérapeutique selon l'une quelconque des revendications 16 à 28, dans laquelle l'hydrolysat de protéine comprend une protéine résistante.

30. La composition thérapeutique selon l'une quelconque des revendications 16 à 29, dans laquelle l'hydrolysat de protéine a un poids moléculaire de 300 à 100 000 D, de préférence de 500 à 50 000 D, plus préférentiellement de 500 à 30 000 D, encore plus préférentiellement de 900 à 30 000 D et de manière préférée de 1 600 à 30 000 D.

31. La composition thérapeutique selon l'une quelconque des revendications 16 à 30, dans laquelle le degré d'hydrolyse de la protéine est de 1 à 40%, de préférence de 3 à 30% et de manière préférée de 5 à 20%.

32. La composition thérapeutique selon l'une quelconque des revendications 16 à 31, comprenant en outre du stérol végétal.

33. La composition thérapeutique selon la revendication 32, dans laquelle le rapport pondéral stérol végétal:protéine est de 1:0,02 à 1:150, de préférence de 1: 0,2 à 1:30, plus préférentiellement de 1:0,4 à 1:12,5 et de manière préférée de 1:1 à 1:5.

34. Un produit alimentaire comprenant au moins un ingrédient nutritionnel de base et une composition thérapeutique selon l'une quelconque des revendications 1 à 33.

35. Utilisation d'une composition comprenant selon l'une quelconque des revendications 1 à 33, pour la fabrication d'un produit pharmaceutique ou nutraceutique ou alimentaire pour améliorer le profil lipidique du sérum.

36. Un procédé de préparation d'une composition thérapeutique selon l'une quelconque des revendications 1 à 15, ledit procédé comprenant le traitement d'une fraction d'avoine comprenant à la fois de la protéine que des lipides émulsifiants et des hydrates de carbone en vue d'enlever les hydrates de carbone, de préférence par hydrolyse enzymatique, et obtenir ladite composition thérapeutique ayant une teneur accrue en protéines.

37. Un procédé de préparation d'une composition thérapeutique selon l'une quelconque des revendications 16 à 33, ledit procédé comprenant l'hydrolyse d'une fraction d'avoine comprenant à la fois de la protéine et des lipides émulsifiants pour obtenir ladite composition thérapeutique.

38. Le procédé selon la revendication 37, dans lequel le degré d'hydrolyse est de 1 à 40%, de préférence de 3 à 30% et de manière préférée de 5 à 20%.

39. Le procédé selon la revendication 37 ou 38, dans lequel la fraction d'avoine est hydrolysée par hydrolyse enzymatique, de préférence en utilisant une protéase.
